# EUROPÄISCHE PATENTANMELDUNG

(11) **EP 2 360 262 A1**
(43) Veröffentlichungstag der Anmeldung: **24.08.2011**
(21) Anmeldenummer: 11153204.0
(22) Anmeldetag: 03.02.2011
(51) Int. Cl.: C12P 7/66, C12N 9/10

(54) **Verfahren zur fermentativen Herstellung von Menachinon-7 mit Escherichia coli**

(30) Priorität: 11.02.2010 DE 102010001832
(71) Anmelder: Wacker Chemie AG, 81737 München (DE)
(72) Erfinder: Schlösser, Thomas, 84489, Burghausen (DE)
(74) Vertreter: Potten, Holger

(57) **Zusammenfassung**

Die Erfindung betrifft ein Verfahren zur Herstellung von Menachinon-7, dadurch gekennzeichnet, dass Zellen eines E. coli-Stammes enthaltend das hepS-Gen aus B. *subtilis* DSM 1088, das hepT-Gen aus B. *subtilis* DSM 1088 und das putative Heptaprenyltransferase-Gen aus B. *subtilis* DSM 1088 in einem Fermentationsmedium fermentiert werden und dabei Menachinon-7 in den Zellen des fermentierten E. coli-Stammes angehäuft wird.

SEQUENCE LISTING

<110>  Wacker Chemie AG - Consortium für elektrochemische
       Industrie

<120>  Verfahren zur fermentativen Herstellung von Menachinon-7 mit
       Escherichia coli

<130>  Co10916

<160>  11

<170>  PatentIn version 3.3

<210>  1
<211>  756
<212>  DNA
<213>  Bacillus subtilis DSM 1088

<400>  1
atgcaagaca tctacggaac tttagccaat ctgaacacga aattaaaaca aaagctgtct      60

catccttatt tagcgaagca tatttctgcg ccgaaaattg atgaggataa gcttcttctt     120

tttcatgctt tatttgaaga agctgacata aaaaacaacg acagagaaaa ttatattgta     180

acagcgatgc ttgtacaaag cgcccttgat acccatgatg aagtgacgac agctagagtc     240

ataaaacgag acgaaaacaa aaaccgccaa ttgactgttc tcgcgggcga ttatttcagc     300

gggctgtact actctttact atctgaaatg aaggatatct acatgattcg gacgcttgct     360

acagccatta aagaaatcaa cgaacataaa attcgtctgt atgaccgttc tttcaaggac     420

gaaaacgatt ttttcgaaag tgtcggcatc gttgaatcag ctttattcca tcgtgtggcg     480

gaacacttca gcctcccgcg ctggaaaaag ctgtcgagtg atttttttgt atttaagcgg     540

cttatgaacg gaaatgatgc atttctggat gtgatcggca gttttataca gctgggaaaa 600

acaaaagaag agatattaga agattgtttt aaaaaagcga aaaacagcat tgagtcactt 660

ctgcctctaa attcacctat tcagaacatt ttaataaacc gtctgaagac aatcagccaa 720

gatcaaacct atcatcagaa agtggaagaa gggtaa 756

<210> 2
<211> 1047
<212> DNA
<213> Bacillus subtilis DSM 1088

<400> 2
atgttaaata tcattcgttt actggcggag tcgctgccac gcatatcgga tggaaatgaa 60

aacacagatg tttgggtgaa tgatatgaaa tttaaaatgg cctactcttt tttaaatgac 120

gatattgatg taatcgaaag agaacttgaa caaaccgtac gttccgatta cccgctttta 180

agcgaggcag gtcttcacct gctgcaggcc ggagggaaac gtattcgtcc tgttttcgtg 240

ctgctttctg gcatgtttgg cgattacgat attaataaga ttaaatatgt cgccgtcact 300

ctggaaatga ttcacatggc atctttggtt catgatgatg tcattgatga tgcagagctt 360

cgccgaggaa aaccgacaat caaagcaaag tgggacaatc gtattgcgat gtacacaggc 420

gattatatgc ttgcgggatc tcttgaaatg atgacgagaa ttaacgaacc gaaagcccat 480

aggattttgt cacagacgat cgttgaagtt tgtctagggg aaattgagca gatcaaagac 540

aaatataaca tggaacaaaa tctcagaacg tatctccgcc gtatcaaaag aaaaacagct 600

ctcttgatcg cggtcagctg ccagcttggt gccattgcgt ctggagctga tgagaagatt 660

cataaggcat tgtactggtt tgggtattac gtcggcatgt cttatcagat tattgatgat 720

attcttgatt ttacttcaac tgaagaagag ctgggtaaac ccgttggagg agatttgctt 780

caaggaaacg tcacattgcc agtgctgtat gccctgaaaa atcctgcatt aaaaaaccag 840

cttaaattga ttaacagtga gacaacgcag gagcagcttg aaccaatcat tgaagaaatc 900

aaaaaaacag atgcaattga agcatctatg gcagtaagcg aaatgtatct gcagaaagct 960

tttcagaaat taaacacgct tccacgaggg cgcgcacgct cgtctcttgc agccatcgca 1020

aaatatatcg gtaaaagaaa attttaa 1047

<210> 3
<211> 936
<212> DNA
<213> Bacillus subtilis DSM 1088

<400> 3
atgaaccaaa caaataaggg tgagggtcag acagcgccgc aaaaagaaag catggggcag 60

atcctttggc agttaacccg tcctcatacg ttaaccgcat cgtttgtgcc tgtgctgctc 120

ggaaccgttt tggcgatgtt ttatgtgaag gttgatctgc tgctgttttt ggctatgctg 180

ttttcttgcc tatggatcca gatcgcgacg aacttattta atgaatatta tgattttaaa 240

cgcggattag atacagcaga atcagtcgga atcggagggg caattgtacg ccacggaatg 300

aagcctaaaa cgattttgca attagctctg gcatcatacg ggattgccat tttgctcggt 360

gtctatattt gtgcgagcag cagctggtgg cttgcgctga tcggccttgt cggcatggcg 420

atcggctacc tgtatacagg cgggccgctg ccgattgcgt acacgccgtt cggtgaatta 480

ttctcaggca tttgcatggg ttcggtgttt gtgctgattt cgtttttcat tcagacagat 540

atgatcaaca cgcaaagcat tttgatttcc atcccgattg cgattcttgt cggcgcgatt 600

aatttgtcaa acaacattcg cgatattgaa gaggacaaaa aaggcggccg caaaacattg 660

gcgattttga tggggcataa gggagctgtt actctgttag ctgcgtcgtt tgccgtcgct 720

tatatctggg ttgtcggctt ggttattacc ggtgccgcaa gcccatggct gtttgtcgtc 780

tttttgagcg tgcctaagcc ggttcaggca gtgaagggct tcgtccaaaa agaaatgccg 840

atgaatatga ttgtcgcaat gaaatcaaca gcccaaacaa atacattttt cggattcctg 900

ctttcgatcg gattattgat cagctatttc cgataa 936

<210> 4
<211> 39
<212> DNA
<213> Artificial Sequence

<220>
<223> Oligonukleotide

<400> 4
aaagggccca ggaggtagaa tgcaagacat ctacggaac 39

<210> 5
<211> 29
<212> DNA
<213> Artificial Sequence

<220>
<223> Oligonucleotide

<400> 5
ccccttaagt tacccttctt ccactttct 29

<210> 6
<211> 39
<212> DNA
<213> Artificial Sequence

<220>
<223> Oligonucleotide

<400> 6
aaacttaaga ggaggtagaa tgttaaatat cattcgttt 39

<210> 7
<211> 29
<212> DNA
<213> Artificial Sequence

<220>
<223> Oligonucleotide

<400> 7
cccagatctt taaaattttc ttttaccga 29

<210> 8
<211> 39
<212> DNA
<213> Artificial Sequence

<220>
<223> Oligonucleotide

<400> 8
aaaagatcta ggaggtagaa tgaaccaaac aaataaggg 39

<210> 9
<211> 31
<212> DNA
<213> Artificial Sequence

<220>
<223> Oligonucleotide

<400> 9
cccttaatta attatcggaa atagctgatc a 31

<210> 10
<211> 35
<212> DNA
<213> Artificial Sequence

<220>
<223> Oligonucleotide

<400> 10
aaagagctcg aattcgcata cgcgtgaggc gagtc 35

<210> 11
<211> 29
<212> DNA
<213> Artificial Sequence

<220>
<223> Oligonucleotide

<400> 11
aaagggcccc tgaatttcat cttttgtaa 29

## Beschreibung

Die Erfindung betrifft ein Verfahren zur fermentativen Herstellung von Menachinon-7 mittels Escherichia *coli.*

Menachinon-7 gehört zur Vitamin K-Familie. Die Hauptquelle für Menachinone bei der Ernährung des Menschen sind im Darm lebende Bakterien, wie beispielsweise *Lactobacillus acidophilus* oder *Escherichia coli.* Biochemisch sind Menachinone an der Carboxlierung von spezifischen Glutaminsäureresten zu γ - Carboxy-glutaminsäure (GLA) beteiligt. Ein prominentes Protein mit 3 GLA-Resten ist Osteocalcin. Osteocalcin wird in den Osteoblasten gebildet und macht 15-20 % der Nicht-Kollagen-Proteine im Knochen aus. Vitamin K-Mangel führt zu nicht carboxyliertem osteocalcin, das ins Plasma gelangt und ein wichtiger Indikator für Störungen des Knochenstoffwechsels ist.

Studien zeigen, dass eine ergänzende Zufuhr von Menachinon-7 beim Menschen sowohl positive Effekte auf die Knochenbildung, als auch eine vorbeugende Wirkung gegenüber Arterienverkalkung besitzt.

Eine Studie zeigt beispielsweise, dass eine verminderte Vitamin K-Zufuhr mit verringerter Knochendichte und einem erhöhten Risiko für Hüftfrakturen assoziiert ist (Feskanich et al., 1999, Am. J. Clin. Nutr. 69: 74-79).

In einer weiteren Studie wird gezeigt, dass eine Supplementierung der Nahrung mit Menachinon-7 zu einer verbesserten γ-Carboxylierung von Osteocalcin führt (Tsukamoto, 2004, BioFactors 22: 5-19).

Traditionell wird zur Herstellung von Menachinon-7 ein Bacillus *subtilis-*Stamm verwendet, der natürlicherweise einen höheren Gehalt von Menachinon-7 aufweist. Dieser Stamm wird im asiatischen Raum zur Herstellung von Natto eingesetzt und hat daher auch die Bezeichnung *Bacillus* subtilis natto oder Bacil*lus natto* (Earl et al., 2007, J. Bacteriol. 189: 1163-1170). Eine Herstellungsmöglichkeit von Menachinon-7 ist daher die Fermentation von Sojabohnen mit Hilfe von *Bacillus* subtilis *natto* und anschließender Islolierung von Menachinon-7 aus dem Natto. EP1803820B1 beschreibt als alternative Herstellungsmöglichkeit die industrielle Fermentation eines Bacillus subtilis-Stammes, der Menachinon-7 produziert. Der Stamm kann anschließend direkt sprühgetrocknet und als Menachinon-7-haltige Biomasse verkauft werden.

Aufgrund der vorbeugenden Eigenschaften gegenüber Osteoporose und Arterienverkalkung werden zunehmend Lebensmittel mit Vitamin K angeboten. Der Zusatzstoff kann entweder dem Nahrungsmittel zugesetzt werden, wie es beispielsweise für Menachinon-4 beschrieben ist, oder es können Menachinon-produzierende Milchsäurebakterien direkt in Lebensmitteln enthalten sein (EP1153548B1 und EP2076585). Milchsäurebakterien wie Lactococ*cus lactis* (Subspecies *cremoris* oder *lactis)* oder *Leuconostoc lactis* produzieren hauptsächlich Menachinon-8 und Menachinon-9 (Morishita et al., 1999, J. Dairy Sci. 82: 1897-1903).

Bakterien im Allgemeinen produzieren Menachinone mit unterschiedlichen Isoprenoid-Kettenlängen und können über diesen Metaboliten teilweise auch taxonomisch unterschieden werden. Bacillus subtilis produziert, wie oben bereits beschrieben, hauptsächlich Menachinon-7.

Im Gegensatz zu *Bacillus subtilis* findet man bei Escherichia *coli* überwiegend die Menachinone Menachinon-8 und Menachinon-9 (Collins und Jones, 1981, Microbiological Reviews 45: 316-354). Die Gene der an der Menachinon-Biosynthese beteiligten Proteine sind bei *Escherichia coli* alle bekannt. Ausgehend von den Ausgangsmetaboliten Chorismat, einer Zwischenstufe der Tryptophan-Biosynthese, und Isoprenylpyrophosphat (IPP), ein zentraler Metabolit der Isoprenoid-Biosynthese, wird zunächst das Intermediat 1,4-Dihydroxy-2-naphtolsäure (DHNA) gebildet. Daran sind die Enzyme MenF (Isochorismat-Synthase), MenD (2-Succinyl-6-hydroxy-2,4-cyclohexadien-1-carboxylat Synthase), MenC (O-Succinylbenzoat Synthase), MenE (O-Succinylbenzoat-CoA Ligase) und MenB (Naphthoat-CoA Synthase) beteiligt. Über die DNHA-Prenyltransferase, die durch menA codiert wird, wird eine Octaprenylpyrophosphat-Einheit auf DHNA übertragen wobei CO₂ und Pyrophosphat freigesetzt werden. Es entsteht Demethylmenachinon (DMK). Beim letzten Biosyntheseschritt wird DMK durch die S-Adenosylmethionin-abhängige Methyltransferase UbiE methyliert, wodurch Menachinon-8 entsteht. Octaprenylpyrophosphat wird mit Hilfe von drei Enzymen aus IPP synthetisiert, die durch die Gene idi, ispA und ispB codiert werden. In einem ersten Syntheseschritt wird IPP durch die Isopentenyldiphosphat Isomerase Idi isomerisiert und anschließend über zwei IspA-katalysierte Reaktionsschritte zu einer C15-Einheit verlängert. In weiteren vier Reaktionsschritten, die alle durch die Octaprenylsynthase IspB katalysiert werden, wird die C40-Einheit Octaprenylpyrophosphat synthetisiert.

Im Bakterium *Bacillus subtilis* läuft die Menachinon-Biosynthese vergleichbar ab. Allerdings ist die Heptaprenyltransferase, ein Schlüsselenzym, das die Heptaprenyleinheit auf DHNA überträgt, weder genetisch identifiziert noch biochemisch beschrieben. Für einen großvolumigen industriellen Prozess zur Herstellung von Menachinon-7 ist der genetische Zugang zu den Menachinon-7-Biosynthesegenen jedoch unabdingbar.

Aufgabe der vorliegenden Erfindung ist die Bereitstellung eines Verfahrens, welches die Herstellung von Menachinon-7 mit *Escherichia coli* ermöglicht.

Diese Aufgabe wird gelöst durch ein Verfahren, das dadurch gekennzeichnet ist, dass Zellen eines E. *coli*-Stammes enthaltend das hepS-Gen aus *B. subtilis* DSM 1088, das hepT-Gen aus B. *subtilis* DSM 1088 und ein putatives Heptaprenltransferase-Gen aus B. *subtilis* DSM 1088 in einem Fermentationsmedium fermentiert werden und dabei Menachinon-7 in den Zellen des fermentierten E. *coli*-Stammes angehäuft wird.

Der E. coli-Stamm umfasst vorzugsweise mehrere funktionelle Kopien der drei benannten Gene. Bevorzugt umfasst er eine bis 700 Kopien der drei benannten Gene. Besonders bevorzugt umfasst er eine bis 20 Kopien der drei benannten Gene. Die Gene können auf einem oder mehreren Plasmiden vorliegen, sie können jedoch ebenso in das Chromosom integriert sein.

Die Erfindung umfasst daher ebenfalls ein Plasmid enthaltend das hepS-Gen aus B. subtilis DSM 1088, das hepT-Gen aus B. *subtilis* DSM 1088 und ein putatives Heptaprenltransferase-Gen aus B. *subtilis DSM* 1088. Ein derartiges Produktionsplasmid ermöglicht die industrielle Herstellung von Menachinon-7 in *Escherichia coli.*

Die drei Gene sind wie folgt definiert:
Das hepS-Gen aus B. subtilis DSM 1088 ist vorzugsweise charakterisiert durch SEQ ID NO 1 sowie Varianten dieser Sequenz,
die durch den degenerierten genetischen Code bedingt sind oder
die für ein Protein codieren, welches die Funktion einer HepS-Untereinheit in einer Heptaprenylsynthase besitzt. Besonders bevorzugt handelt es sich um SEQ ID NO 1 sowie Varianten dieser Sequenz, die durch den degenerierten genetischen Code bedingt sind.

Das hepT-Gen aus B. *subtilis* DSM 1088 ist vorzugsweise charakterisiert durch SEQ ID NO 2 sowie Varianten dieser Sequenz, die durch den degenerierten genetischen Code bedingt sind oder die für ein Protein codieren, welches die Funktion einer HepT-Untereinheit in einer Heptaprenylsynthase besitzt. Besonders bevorzugt handelt es sich um SEQ ID NO 2 sowie Varianten dieser Sequenz, die durch den degenerierten genetischen Code bedingt sind.

Ein Protein besitzt vorzugsweise dann die Funktion einer Heps-Untereinheit in einer Heptaprenylsynthase, wenn es zusammen mit einem HepT-Protein, codiert durch ein hepT-Gen mit der SEQ ID NO 2 ein Heterodimer bildet, welches eine Heptaprenylsynthase-Aktivität besitzt, die mindestens der Heptaprenylsynthase-Aktivität eines Heterodimers aus HepS, codiert durch ein Gen mit SEQ ID No 1, und HepT, codiert durch ein Gen mit SEQ ID No 2, entspricht.

Ein Protein besitzt vorzugsweise dann die Funktion einer HepT-Untereinheit in einer Heptaprenylsynthase, wenn es zusammen mit einem HepS-Protein, codiert durch das hepS Gen mit der SEQ ID NO 1, ein Heterodimer bildet, welches eine Heptaprenylsynthase-Aktivität besitzt, die mindestens der Heptaprenylsynthase-Aktivität eines Heterodimers aus HepT, codiert durch ein Gen mit SEQ ID No 2, und HepS, codiert durch ein Gen mit SEQ ID No 1, entspricht.

Das putative Heptaprenyltransferase-Gen aus B. subtilis DSM 1088 ist charakterisiert durch SEQ ID NO 3 sowie Varianten dieser Sequenz die durch den degenerierten genetischen Code bedingt sind oder die für ein Protein mit Heptaprenyltransferase-Aktivität codieren. Besonders bevorzugt handelt es sich um SEQ ID NO 3 sowie Varianten dieser Sequenz, die durch den degenerierten genetischen Code bedingt sind.

Die Funktionen der erfindungsgemäßen Heptaprenylsynthasen und -transferasen in E. *coli* können indirekt über das Produkt Menachinon-7 charakterisiert werden, da Wildtyp-Stämme von *Escherichia coli* kein Menachinon-7 produzieren. Als Nachweismethode kann die in Beispiel 5 beschriebene HPLC-Methode verwendet werden.

Vorzugsweise ist zur Herstellung von Menachinon-7 ist die Expression des hepS-Gens aus B. subtilis DSM 1088, des hepT-Gens aus B. *subtilis* DSM 1088 und des putativen Heptaprenyltransferase-Gens aus B. subtilis DSM 1088 auf einem Plasmid in *E.coli* notwendig.

Die Expression dieser Gene wird vorzugsweise durch homologe oder heterologe Promotoren erreicht. Der Begriff heterolog bezieht sich auf die Gene charakterisiert durch die SEQ ID NO 1, SEQ ID NO 2 und SEQ ID NO 3. Entsprechende heterologe Promotoren sind beispielsweise der Promotor des gapA-Gens aus *E. coli* oder der Promotor des tufB-Gens aus *E. coli.* Weiterhin sind die heterologen lac-, tac-, trc-, lambda-, ara- oder tet-Promotoren dem Fachmann bekannt.

Bevorzugt wird die Expression des hepS-Gens aus *B. subtilis* DSM 1088, des hepT-Gens aus *B. subtilis* DSM 1088 und des putativen Heptaprenyltransferase-Gens aus *B. subtilis* DSM 1088 durch den Promotor des gapA-Gens aus *E. coli* erreicht.

Das erfindungsgemäße Plasmid umfasst daher vorzugsweise die oben genannten Promotoren, wobei die Promotoren sich derart auf dem Plasmid befinden, dass sie die Expression des hepS-Gens aus *B. subtilis* DSM 1088, des hepT-Gens aus *B. subtilis* DSM 1088 und des putativen Heptaprenyltransferase-Gens aus *B. subtilis* DSM 1088 steuern.

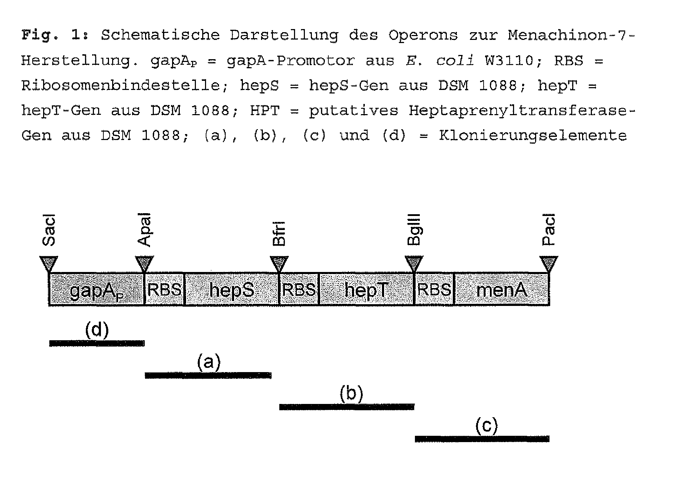

Besonders bevorzugt enthält das Plasmid eine Operon-Konstruktion bei dem das hepS-Gens aus *B. subtilis* DSM 1088, das hepT-Gens aus *B. subtilis* DSM 1088 und das putative Heptaprenyltransferase-Gen aus *B. subtilis* DSM 1088 unter Kontrolle des gapA-Promotors aus *E. coli* stehen. Ein derartiges Konstrukt ist beispielhaft in Fig. 1 wiedergegeben.

Als Promotorregion zur Expression des hepS-Gens aus *B. subtilis* DSM 1088, des hepT-Gens aus *B. subtilis* DSM 1088 und des putativen Heptaprenyltransferase-Gens aus *B. subtilis* DSM 1088 können jedoch auch die natürlichen (homologen) Promotorregionen dieser Gene dienen.

Als Plasmide, in die das hepS-Gens aus *B. subtilis* DSM 1088, das hepT-Gens aus *B. subtilis* DSM 1088 und das putative Heptaprenyltransferase-Gen aus *B. subtilis* DSM 1088 eingebracht werden, können alle verfügbaren und gentechnisch zugänglichen DNS-Moleküle verwendet werden, die extrachromosomal in *Escherichia coli* repliziert werden und die einen Selektionsmarker umfassen. So können beispielsweise Plasmide mit einer hohen zellulären Kopienzahl in *E. coli* (z. B. Plasmide der pUC-Serie, Plasmide der pQE-Serie, Plasmide der pBluescript-Serie), Plasmide mit einer mittleren Kopienzahl in E. *coli* (z. B. Plasmide der pBR-Serie, Plasmide der pACYC-Serie) oder Plasmide mit einer niedrigen Kopienzahl in E. *coli* (z. B. pSC101 oder pBeloBAC11) eingesetzt werden.

Bevorzugt werden Plasmide mit einer mittleren Kopienzahl in E. *coli* (z. B. Plasmide der pBR-Serie, Plasmide der pACYC-Serie verwendet.

Besonders bevorzugt wird ein Plasmid der pACYC-Serie verwendet.

Zur Produktion von Menachinon-7 wird ein erfindungsgemäßes Plasmid in einen *Escherichia coli* Stamm eingebracht.

Dies geschieht beispielsweise durch eine gängige Transformationsmethode wie z.B. Elektroporation oder CaCl₂-Methode. Plasmid-tragende Klone werden anschließend über eine Antibiotika-Resistenz selektiert. Dem Fachmann bekannte Selektionsmarker sind beispielsweise das Chloramphenicol-Acetyltransferase-Gen, das Resistenz gegenüber Chloramphenicol vermittelt, das Neomycin-Phosphotransferase-Gen, das Resistenz gegenüber Kanamycin vermittelt, das Tetracyclin-Efflux-Gen, das Resistenz gegenüber Tetracyclin vermittelt oder das β-Lactamase-Gen, das Resistenz gegenüber Ampicillin und Carbenicillin vermittelt.

Alternativ zur Expression von einem erfindungsgemäßen Plasmid können das hepS-Gens aus *B. subtilis* DSM 1088, das hepT-Gens aus *B. subtilis* DSM 1088 und das putative Heptaprenltransferase-Gen aus *B. subtilis* DSM 1088 auch in das Chromosom eines E. *coli*-Stammes zusätzlich oder im Austausch zu den bereits vorhandenen Menachinon-Biosynthesegenen integriert werden. Als Integrationsverfahren werden vorzugsweise die dem Fachmann bekannten Systeme mit temperenten Bakteriophagen, integrativen Plasmiden oder die Integration über homologe Rekombination genutzt.

Die Erfindung betrifft daher auch *E. coli* Stämme, die ein erfindungsgemäßes Plasmid oder mehrere, vorzugsweise je eine bis je fünf chromosomale Kopien des hepS-Gens aus B. *subtilis* DSM 1088, des hepT-Gens aus B. *subtilis* DSM 1088 und des putativen Heptaprenyltransferase-Gens aus B. *subtilis* DSM 1088 enthalten.

Die Produktion von Menachinon-7 mit Hilfe eines erfindungsgemäßen E. *coli*-Stammes erfolgt in einem Fermenter nach an sich bekannten Verfahren.

Die Anzucht des E. *coli*-Stammes im Fermenter erfolgt als kontinuierliche Kultur, als Batch-Kultur oder vorzugsweise als Fed-Batch-Kultur.

Als Kohlenstoff-Quelle dienen vorzugsweise Zucker, Zuckeralkohole oder organische Säuren. Besonders bevorzugt werden im erfindungsgemäßen Verfahren als Kohlenstoff-Quellen Glucose, Lactose, Saccharose oder Glycerin eingesetzt.

Bevorzugt ist die Dosierung der Kohlenstoff-Quelle in einer Form, die gewährleistet, dass der Gehalt an Kohlenstoff-Quelle im Fermenter während der Fermentation in einem Bereich von 0,1 g/L bis 50 g/L gehalten wird. Besonders bevorzugt ist ein Bereich von 0,1 g/L bis 10 g/L.

Als Stickstoff-Quelle werden im erfindungsgemäßen Verfahren vorzugsweise Ammoniak, Ammoniumsalze oder Proteinhydrolysate verwendet. Bei Verwendung von Ammoniak als Korrekturmittel zur pH-Kontrolle wird während der Fermentation regelmäßig diese Stickstoff-Quelle nachdosiert.

Als weitere Medienzusätze können Salze der Elemente Phosphor, Chlor, Natrium, Magnesium, Stickstoff, Kalium, Calcium, Eisen und in Spuren (d.h. in µM Konzentrationen) Salze der Elemente Molybdän, Bor, Kobalt, Mangan, Zink, Kupfer und Nickel zugesetzt werden.

Des Weiteren können organische Säuren (z.B. Acetat, Citrat), Aminosäuren (z.B. L-Isoleucin, D/L-Methionin) und Vitamine (z.B. Vitamin B1, Vitamin B6, Vitamin B12) dem Medium zugesetzt werden.

Als komplexe Nährstoffquellen können z.B. Hefeextrakt, Maisquellwasser, Sojamehl oder Malzextrakt zum Einsatz kommen.

Die Inkubationstemperatur für *Escherichia coli* beträgt vorzugsweise 28 - 37 °C, besonders bevorzugt ist eine Inkubationstemperatur von 30 - 32 °C.

Der pH-Wert des Fermentationsmediums liegt während der Fermentation bevorzugt im pH-Bereich von 5,0 bis 8,5, besonders bevorzugt ist ein pH-Wert von 7,0.

Die Inkubation des E. *coli*-Stammes erfolgt unter aeroben, anaeroben oder mikroaeroben Kultivierungsbedingungen über einen Zeitraum von 16 h bis 150 h und im Bereich der für den jeweiligen Stamm optimalen Wachstumstemperatur. Besonders bevorzugt sind Kultivierungszeiten zwischen 48 h und 96 h.

Die Fermentation wird vorzugsweise unter aeroben oder mikroaeroben Wachstumsbedingungen durchgeführt.

Die Abtrennung von Menachinon-7 aus der Kultur kann nach dem Fachmann bekannten Verfahren, wie Zentrifugation des Mediums zur Abtrennung der Zellen mit anschließender Extraktion des Menachinon-7 aus der Biomasse, chromatographischer Reinigung, Konzentrierung, Formulierung oder Komplexierung des Produktes, erfolgen.

Der Nachweis und die Quantifizierung des im erfindungsgemäßen Verfahren produzierten Menachinon-7 erfolgt beispielsweise mittels einer HPLC-Methode.
Fig. 1 zeigt, schematisch dargestellt, ein erfindungsgemäßes Operon-Konstrukt.
Fig. 2 zeigt schematisch ein zur Herstellung von Menachinon-7 geeignetes Plasmid pKG82.

Die folgenden Beispiele dienen der weiteren Erläuterung der Erfindung.

### Beispiel 1: Konstruktion des Plasmids pKG82

a) Amplifizierung des hepS-Gens:
   Das hepS-Gen aus *Bacillus subtilis* (DSM 1088) wurde mittels der Polymerase-Ketten-Reaktion (PCR) unter Verwendung der Taq-DNS-Polymerase (Roche, Mannheim) nach gängiger, dem Fachmann bekannter Praxis amplifiziert. Als Matrize diente die chromosomale DNS des *Bacillus* subtilis-Stammes DSM 1088. Als Primer wurden die Oligonukleotide M7-Operon-hepS-for (SEQ ID NO 4) und M7-Operon-hepS-rev (SEQ ID NO 5) verwendet.
   Das bei der PCR erhaltene DNS-Fragment mit einer Länge von 783 Basenpaaren wurde anschließend mittels eines DNS-Adsorptionssäulchens des QIAprep Spin Miniprep Kits (Qiagen, Hilden) nach Herstellerangaben gereinigt.
b) Amplifizierung des hepT-Gens:
   Das hepT-Gen aus *Bacillus subtilis* (DSM 1088) wurde mittels der Polymerase-Ketten-Reaktion (PCR) unter Verwendung der Taq-DNS-Polymerase (Roche, Mannheim) nach gängiger, dem Fachmann bekannter Praxis amplifiziert. Als Matrize diente die chromosomale DNS des *Bacillus subtilis*-Stammes DSM 1088. Als Primer wurden die Oligonukleotide M7-Operon-hepT-for (SEQ ID NO 6) und M7-Operon-hepT-rev (SEQ ID NO 7) verwendet.
   Das bei der PCR erhaltene DNS-Fragment mit einer Länge von 1075 Basenpaaren wurde anschließend mittels eines DNS-Adsorptionssäulchens des QIAprep Spin Miniprep Kits (Qiagen, Hilden) nach Herstellerangaben gereinigt.
c) Amplifizierung des putativen Heptaprenyltransferase-Gens: Das putative Heptaprenyltransferase-Gen aus *Bacillus subtilis* (DSM 1088) wurde mittels der Polymerase-Ketten-Reaktion (PCR) unter Verwendung der Taq-DNS-Polymerase (Roche, Mannheim) nach gängiger, dem Fachmann bekannter Praxis amplifiziert. Als Matrize diente die chromosomale DNS des *Bacillus* subtilis-Stammes DSM 1088. Als Primer wurden die Oligonukleotide M7-Operon-pHPTG-for (SEQ ID NO 8) und M7-Operon-pHPTG-rev (SEQ ID NO 9) verwendet.
   Das bei der PCR erhaltene DNS-Fragment mit einer Länge von 966 Basenpaaren wurde anschließend mittels eines DNS-Adsorptionssäulchens des QIAprep Spin Miniprep Kits (Qiagen, Hilden) nach Herstellerangaben gereinigt.
d) Amplifizierung des gapA-Promotors aus E. *coli:*
   Der gapA-Promotor aus E. *coli* W3110 (ATCC 27325) wurde mittels der Polymerase-Ketten-Reaktion (PCR) unter Verwendung der Taq-DNS-Polymerase (Roche, Mannheim) nach gängiger, dem Fachmann bekannter Praxis amplifiziert. Als Matrize diente die chromosomale DNS des *E*. *coli*-Stammes W3110 (ATCC 27325). Als Primer wurden die Oligonukleotide M7-Operon-gapA-for (SEQ ID NO 10) und M7-Operon-gapA-rev (SEQ ID NO 11) verwendet.
   Das bei der PCR erhaltene DNS-Fragment mit einer Länge von 319 Basenpaaren wurde anschließend mittels eines DNS-Adsorptionssäulchens des QIAprep Spin Miniprep Kits (Qiagen, Hilden) nach Herstellerangaben gereinigt.
e) Klonierung der Gene hepS, hepT und des putativen Heptaprenyltransferase-Gens in den Plasmid-Vektor pACYC184-LH unter gapA-Promotor-Kontrolle:
   Die DNS-Fragmente aus a), b), c) und d) wurden vor dem Ligationsansatz mit T4-DNS-Ligase (Roche, Mannheim) mit folgenden Restriktionsendunucleasen (Roche, Mannheim) geschnitten:
      (I): Fragment aus a) mit SacI und ApaI
      (II): Fragment aus b) mit ApaI und BfrI
      (III): Fragment aus c) mit BfrI und BglII
      (IV): Fragment aus d) BglII und PacI (New England Biolabs,

### Frankfurt am Main).

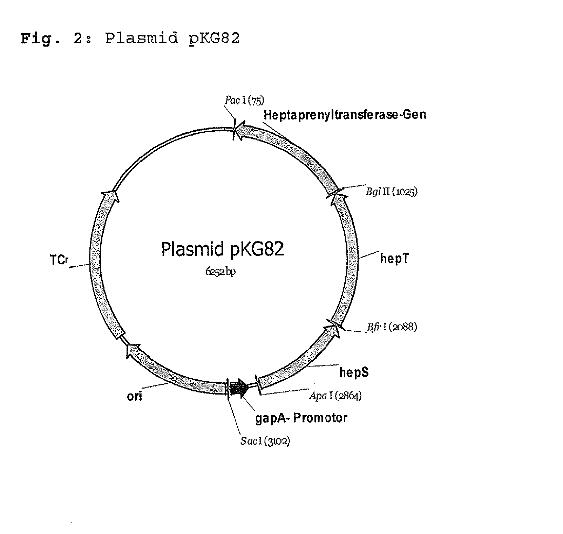

Der Klonierungsvektor pACYC184-LH (DSM 10172) wurde mit den Restriktionsendonucleasen SacI (Roche, Mannheim) und PacI (New England Biolabs, Frankfurt am Main) geschnitten und anschließend nach erfolgter Reinigung mit den geschnitten Fragmenten aus (I), (II), (III) und (IV) in einem Ligationsansatz ligiert (siehe Fig. l). Das daraus resultierende Plasmid wurde durch Sanger-Sequenzierung auf seine richtige Identität hin überprüft. Das fertige Menachinon-7-Produktionsplasmid wurde mit pKG82 bezeichnet (siehe Fig. 2).

Das Plasmid pKG82, das für Produktion von Menachinon-7 verwendet wurde, wurde am 27.11.2009 bei der DSMZ (Deutsche Sammlung für Mikroorganismen und Zellkulturen GmbH, D-38142 Braunschweig) unter der Nummer DSM 23159 gemäß Budapester Vertrag hinterlegt.

### Beispiel 2: Herstellung eines Menachinon-7-Produktionsstammes

Das in Beispiel 1 beschriebene Plasmid pKG82 wurde mittels CaCl₂-Methode zur Transformation des E. *coli*-Stammes W3110 (ATCC 27325) eingesetzt. Nach Selektion auf LB-Agarplatten mit 20 mg/L Tetracyclin wurde das Plasmid aus einer der Transformanten reisoliert, mit Restriktionsendonucleasen gespalten und überprüft. Dieser Stamm wird als W3110/pKG82 bezeichnet und ist für die Produktion von Menachinon-7 geeignet.

### Beispiel 3: Erste Vorkultur (Tageskultur) eines Menachinon-7-Produktionsstammes für die Fermentation

20 mL LB-Medium mit Glucose (10 g/L Trypton, 5 g/L Hefeextrakt, 5 g/L NaCl, 15 g/L Glucose; autoklaviert) wurden in einem sterilen 100 mL Erlenmeyerkolben mit Tetracyclin x HCl versetzt (Endkonzentration von Tetracyclin x HCl: 15 mg/L; Stammlösung von Tetracyclin x HCl: 10 mg/mL in 50% Ethanol sterilfiltriert).
Mit einer Impföse wurden soviel Zellen des Produktionsstammes von der Agarplatte abgenommen bis maximal eine schwache Trübung erkennbar wurde. Die Vorkulturen wurden für 8 h bei 32°C (Stämme W3110 und W3110/pKG82) bzw. 30 °C (Stamm DSM 1088) und 150 rpm kultiviert.

### Beispiel 4: Zweite Vorkultur (Übernachtkultur) eines Menachinon-7-Produktionsstammes für die Fermentation

Die zweite Vorkultur (Übernachtkultur) erfolgte in einem 2 L-Fermenter mit Hilfe eines Biostat®-B-DCU-Geräts der Firma B. Braun Biotech International (Melsungen, Deutschland). 20 mL LB-Vorkultur aus Beispiel 3 dienten zum Animpfen der 980 mL Produktionsmedium, bestehend aus 25 g/L Glucose, 0,015 g/L Thiamin x HCl, 0,015 g/L Tetracyclin x HCl, 3 g/L (NH₄)₂SO₄, 0,25 g/L NaCl, 0,9 g/L L-Isoleucin, 0,6 g/L D/L-Methionin, 30 g/L Cornsteep Liquor (Roquette, Lestrem, Frankreich), 0, 03 g/L CaCl₂ x 2 H₂O, 0, 6 g/L MgSO₄ x 7 H₂O, 0,15 g/L Na₂MO₄ x 2 H₂O, 2,5 g/L H₃BO₃, 0,7 g/L CoCl₂ x 6 H₂O, 0, 25 g/L CuSO₄ x 5 H₂O, 1,6 g/L MnCl₂ x 4 H₂O, 0, 3 g/L ZnSO₄ x 7 H₂O, 0,15 g/L FeSO₄ x 7 H₂O, 1 g/L Na₃-Citrat x 2 H₂O und 1,7 g/L KH₂PO₄. Die Medienbestandteile wurden steril in einem 2 L-Fermenter vorgelegt. Während der Kultivierung für 17 h wurden die Temperatur auf 32 °C (Stämme W3110 und W3110/pKG82) oder 30 °C (Stamm DSM 1088) sowie der pH-Wert bei 7,0 durch das Korrekturmittel (25 % Ammoniak) konstant gehalten. Als Antischaummittel wurde Struktol J673 (Schill + Seilacher, Hamburg) in einer Verdünnung von 1:6 in sterilem Wasser verwendet. Die Kultur wurde mit keimfreier Druckluft bei 5 vol/vol/min begast und mit einer Rührerdrehzahl von 400 rpm gerührt. Nach Absinken der Sauerstoffsättigung auf einen Wert von 50 % wurde die Drehzahl über die Steuerungseinheit des Fermenters bis zu einem Wert von 1500 rpm erhöht um 50 % Sauerstoffsättigung zu erhalten. Die Sauerstoffsättigung wurde mit einer PO₂-Sonde bestimmt, die bei 400 rpm auf 100% Sättigung kalibriert wurde. Sobald der Glucose-Gehalt im Fermenter auf ca. 5 g/L abgesunken war, erfolgte die Zudosierung einer 56 % (w/v) Glucoselösung. Die Glucose-Fütterung erfolgte mit einer Flussrate von 6-12 mL/h wobei die Glucosekonzentration im Fermenter zwischen 0,1 g/L und 10 g/L konstant gehalten wurde. Die GlucoseBestimmung wurde mit dem Analysator YSI 7100 MBS (YSI, Yellow Springs, Ohio, USA) durchgeführt.

### Beispiel 5: Fermentative Herstellung von Menachinon-7

Die Herstellung von Menachinon-7 erfolgte im Fermenter mit Hilfe eines Biastat^{®}-B-DCU-Geräts der Firma B. Braun Biotech International (Melsungen, Deutschland).
Der Hauptfermenter wurde mit 100 mL Kultur aus einer Übernachtkultur (siehe Beispiel 4) angeimpft. Die Fermentationsbedingungen entsprachen denen des Vorfermenters aus Beispiel 4. Die Fermentationsdauer betrug 96 h. Die Bestimmung von Menachinon-7 erfolgte wie in Suvarna et al. (2008, Journal of Bacteriology 180, Nr. 10, Seiten 2782-2787) beschrieben. Die Probenentnahmen erfolgten jeweils nach 24 h, 48 h, 72 h und 96 h.

Die Analyse der Chinone wurde an einer HPLC HP 1200 von Agilent (Böblingen, Deutschland) durchgeführt. Als Trennsäule diente eine Luna C18(2) RP-HPLC Säule 5µ (100 mm x 4,6 mm) der Firma Phenomenex (Aschaffenburg, Deutschland). Die Proben wurden isokratisch mit Isopropanol-Acetonitril (1:3 [vol/vol]) bei einer Temperatur von 40°C und einem Fluss von 0,7 mL/min eluiert. Die Chinone wurden mit einem UV-Detektor bei den Wellenlängen 207 nm und 280 nm detektiert.

Die Tabelle 1 zeigt die erzielten Gehalte an Menachinon-7, die mit den verschiedenen Stämmen erhalten wurden.

**Tabelle 1:**

| Stamm | Menachinon-7 [mg/g BTM] | | | |
|---|---|---|---|---|
| | 24 h | 48 h | 72 h | 96 h |
| *Bacillus subtilis* DSM 1088^{[1]} | 0,07 | 0,08 | 0,22 | - ^{[2]} |
| *Escherichia coli* W3110 | - ^{[3]} | -^{[3]} | - ^{[3]} | - ^{[3]} |
| W3110/pKG82 | 0,21 | 0, 84 | 1,19 | 1,59 |

| | | | | |
|---|---|---|---|---|
| BTM = Biotrockenmasse ^{[1]} = Aufgrund der extremen Schaumentwicklung des Stammes musste der Airflow nach 8 h Kultivierung auf 1 vol/vol/min und die Rührerdrehzahl auf 400 rpm begrenzt werden ^{[2]} = Fermentation nach 72 abgebrochen ^{[3]} = nicht nachweisbar | | | | |

## Patentansprüche

1. Verfahren zur Herstellung von Menachinon-7, **dadurch gekennzeichnet, dass** Zellen eines E. *coli*-Stammes enthaltend das hepS-Gen aus B. subtilis DSM 1088, das hepT-Gen aus B. subtilis DSM 1088 und das putative Heptaprenyltransferase-Gen aus B. subtilis DSM 1088 in einem Fermentationsmedium fermentiert werden und dabei Menachinon-7 in den Zellen des fermentierten E. coli-Stammes angehäuft wird.

2. Menachinon-7-Produktionsplasmid zur industriellen Herstellung von Menachinon-7 mit *Escherichia* coli, **dadurch gekennzeichnet, dass** es das hepS-Gen aus B. subtilis DSM 1088, das hepT-Gen aus *B. subtilis* DSM 1088 und das putative Heptaprenyltransferase-Gen aus *B. subtilis* DSM 1088 enthält.

3. Menachinon-7-Produktionsplasmid gemäß Anspruch 2, **dadurch gekennzeichnet, dass** es homologe oder heterologe Promotoren enthält.

4. Menachinon-7-Produktionsplasmid gemäß Anspruch 3, **dadurch gekennzeichnet, dass** der heterologe Promotor der Promotor des gapA-Gens aus E. *coli* oder der Promotor des tufB-Gens aus E. *coli* oder der lac-, tac-, trc-, lambda-, ara- oder tet-Promotor ist.

5. Menachinon-7-Produktionsplasmid gemäß Anspruch 2 bis 4,
**dadurch gekennzeichnet, dass** es ein Operon-Konstrukt umfasst, bei dem das hepS-Gen aus B. *subtilis* DSM 1088, das hepT- Gen aus B. *subtilis* DSM 1088 und das putative Heptaprenyltransferase-Gen aus *B. subtilis* DSM 1088 unter Kontrolle des gapA-Promotors aus *E*. coli stehen.

6. Menachinon-7-Produktionsplasmid gemäß Anspruch 2 bis 5,
**dadurch gekennzeichnet, dass** als Plasmid ein DNS-Molekül verwendet wird, das extrachromosomal in *Escherichia coli* repliziert werden kann und das einen Selektionsmarker umfasst.

7. Menachinon-7-Produktionsplasmid gemäß Anspruch 6, **dadurch gekennzeichnet, dass** ein Plasmid mit einer hohen zellulären Kopienzahl in E. *coli* oder ein Plasmid mit einer mittleren Kopienzahl in *E*. coli oder ein Plasmid mit einer niedrigen Kopienzahl in E. *coli* verwendet wird.

8. E. *coli* Stamm enthaltend ein Plasmid gemäß Anspruch 2 bis 7 oder mehrere chromosomale Kopien des hepS-Gens aus B. subtilis DSM 1088, des hepT- Gens aus B. subtilis DSM 1088 und des putativen Heptaprenyltransferase-Gens aus B. *subtilis* DSM 1088.

9. Verfahren gemäß Anspruch 1, **dadurch gekennzeichnet, dass** nach der Fermentation eine Abtrennung von Menachinon-7 mittels Zentrifugation des Fermentationsmediums zur Abtrennung der Zellen und anschließend eine Extraktion des Menachinon-7 aus den Zellen, eine chromatographische Reinigung, eine Konzentrierung, eine Formulierung oder eine Komplexierung des Menachinon-7 erfolgt.
